# EUROPEAN PATENT SPECIFICATION

(11) **EP 3 378 514 B1**
(45) Date of publication and mention of the grant of the patent: **10.09.2025**
(21) Application number: 16866367.2
(22) Date of filing: 17.11.2016
(51) Int. Cl.: A61M 5/31, A61M 5/28

(54) **SYRINGE BARREL, PREFILLED SYRINGE, AND MANUFACTURING METHODS FOR SAME**
SPRITZENZYLINDER, VORGEFÜLLTE SPRITZE UND HERSTELLUNGSVERFAHREN DAFÜR
CYLINDRE POUR SERINGUE, SERINGUE PRÉREMPLIE, ET PROCÉDÉS DE FABRICATION DE CEUX-CI

(30) Priority: 19.11.2015 JP 2015226646
(43) Date of publication of application: 26.09.2018
(73) Proprietor: Terumo Kabushiki Kaisha, Tokyo 151-0072 (JP)
(72) Inventor: OKIHARA, Hitoshi, Fujinomiya-shi Shizuoka 418-0004 (JP)
(74) Representative: Casalonga
(86) International application number: PCT/JP2016/084025
(87) International publication number: WO 2017/086366

(56) References cited:
- EP-A2- 2 061 529
- WO-A1-2010/064667
- WO-A1-2011/052115
- WO-A2-2009/053947
- JP-A- 2000 005 309
- JP-A- 2007 216 019
- JP-A- 2010 503 492
- US-A1- 2011 276 005

## Description

### Technical Field

The present invention relates to a syringe barrel, and methods of manufacturing said syringe barrel.

### Background Art

A typical syringe includes a hollow barrel, a gasket formed of an elastic body slidably inserted in the barrel, and a pusher connected to the gasket, and is configured to dispense a drug solution filled in the barrel with operation of the pusher in the axial direction. In addition, it is conventionally known to spray a silicone oil to be applied to the barrel inner surface so as to suppress the sliding resistance of the gasket in the barrel.

In order to effectively reduce the sliding resistance of the gasket in the barrel, it is effective to uniformly apply the silicone oil to the barrel inner surface. For this reason, for example, JP 2002-143300 A applies a silicone oil by spraying the oil toward the barrel inner surface and then spreading and smoothing the oil with a tool such as a squeegee so as to uniformly apply the silicone oil. Further relevant prior art can be found under EP2061529B1, US2011/0276005A1, and WO2009/053947A2.

### Summary of Invention

In the case of spreading and smoothing the silicone oil applied to the barrel inner surface with a tool as disclosed in JP 2002-143300 A, the barrel inner surface is rubbed with the tool, leading to a possibility of generation of a scratch or adhesion of foreign matter on the barrel inner surface.

The present invention has been made in view of this problem, and aims to provide a syringe barrel, having the silicone oil uniformly applied without generation of scratches or adhesion of foreign matter on the barrel inner surface, and methods for manufacturing these.

To achieve the above object, a method for manufacturing a syringe barrel according to the present invention includes: a providing step of providing a syringe barrel including a hollow body portion and being configured that a gasket sliding on an inner surface of the body portion is inserted therein; an application step of applying a non-reactive silicone oil having a viscosity of 3000 cps to 30000 cps to the inner surface by spraying with a coating amount rate of 0.02 mg/cm² to 0.2 mg/cm²; and a heating step of heating the silicone oil applied to the inner surface without spreading or smoothing, and being heated by performing autoclave sterilization on the syringe barrel to reduce the viscosity so as to allow the silicone oil to spread out in a direction of the inner surface of the body portion and the directions of gravity, the silicone oil applied to the inner surface in a state where a liquid is not filled in the syringe barrel.

With this method for manufacturing a syringe barrel, the non-reactive silicone oil applied to the barrel inner surface is heated to reduce the viscosity, so as to allow the silicone oil to spread out in a direction of the barrel inner surface and the direction of gravity. This increases uniformity of the silicone oil distribution, making it possible to suppress the sliding resistance of the gasket inserted into the syringe barrel. In addition, application of the silicone oil having the above-described viscosity makes it possible to suitably reduce the viscosity at the time of heating and effectively uniformize the silicone oil distribution. Furthermore, application of the silicone oil at the above-described coating amount rate enables the silicone oil to uniformly spread over the barrel inner surface without causing excessive application. In addition, since the silicone oil applied to the barrel inner surface is not spread and smoothed with a tool such as a squeegee to achieve uniformity in the silicone oil distribution, it is possible to obtain a syringe barrel on which the silicone oil is uniformly applied without generation of scratches or adhesion of foreign matter on the barrel inner surface.

In the method for manufacturing a syringe barrel as described above, in the heating step, the silicone oil is heated by performing autoclave sterilization on the syringe barrel.

With this method, the silicone oil is heated at the time of autoclave sterilization for the syringe barrel, making it possible to efficiently manufacture the syringe barrel without a need to provide a dedicated step for heating the silicone oil. In addition, autoclave sterilization can suppress degradation of the syringe barrel as compared with other sterilization treatments such as electron beam sterilization, making it possible to suppress adverse effects on the drug solution filled in the syringe barrel.

In the above-described method for manufacturing a syringe barrel, the heating step may be performed in a state where an axis of the syringe barrel is substantially vertical.

This enables the silicone oil to spread out in a barrel axial direction due to gravity at the time of heating, leading to achievement of higher uniformity in the silicone oil distribution.

In the above-described method for manufacturing a syringe barrel, in the application step, the silicone oil may be applied to the inner surface by spraying the silicone oil from one end side toward the other end side of the syringe barrel, and the heating step may be performed in a state where the other end side faces downward.

With this method, heating is performed with the side opposite to the spraying side facing downward, allowing the mass of the silicone oil to spread out from one end side of the barrel toward the other end side due to gravity. This makes it possible to effectively achieve higher uniformity in the silicone oil distribution.

In the above-described method for manufacturing a syringe barrel, in the application step, the silicone oil may be sprayed from one end side toward the other end side of the syringe barrel, while aspirating air within the syringe barrel from the other end side.

This can suppress unevenness in spraying of silicone oil and achieve application of the silicone oil with higher uniformity.

In the above-described method for manufacturing a syringe barrel, the syringe barrel may include: an opening portion for inserting the gasket at the one end side; and a nozzle having an inner diameter smaller than the inner diameter of the body portion at the other end side.

In this manner, the inner diameter of the nozzle provided on the side opposite to the opening portion of the barrel is smaller than the body portion. Accordingly, the air inside the barrel is not likely to escape from the nozzle side (the other side) when the silicone oil is sprayed from the opening portion (one end side) of the barrel, making it difficult to allow the sprayed silicone oil to reach the nozzle side (the other end side). This makes it more important to spray the silicone oil while aspirating the air from the nozzle side (the other end side) .

In addition, a method for manufacturing a prefilled syringe not according to the present invention includes: a preparation step of preparing the syringe barrel manufactured by any of the methods for manufacturing a syringe barrel;
a filling step of filling the prepared syringe barrel with a drug solution; and a plugging step of plugging the gasket into the syringe barrel filled with the drug solution.

With this method, a prefilled syringe in which silicone oil is uniformly applied to the barrel inner surface is provided.

In the above-described method for manufacturing a prefilled syringe, the drug solution may be a protein preparation.

The silicone oil is uniformly applied with this method, leading to suppression of outflow of the silicone oil into the drug solution, making it possible to suppress the aggregation of the protein caused by the outflow of the silicone oil.

In addition, the present invention is a syringe barrel including: a hollow body portion; a shoulder portion reduced in diameter from a distal end of the body portion; a nozzle protruding in a distal end direction from the shoulder portion; and an opening portion that opens at a proximal end of the body portion, the syringe barrel being configured that a gasket sliding on an inner surface of the bodyportion is inserted therein, in which a non-reactive silicone oil having a viscosity of 3000 cps to 30000 cps is applied on the inner surface at least from an insertion position configured for the gasket being inserted thereat, up to a position closer to the shoulder portion than the insertion position, and a coverage as a ratio of the area of the silicone oil present as droplets to the area of the inner surface is 20% or less at each of sections where a region applied with the silicone oil in the inner surface is partitioned with a predetermined width in the axial direction, and a droplet structure of the silicone oil is present on the inner surface.

With the syringe barrel configured as described above, it is possible to suppress the sliding resistance of the gasket since the silicone oil distribution is uniformized. In addition, since the silicone oil applied to the barrel inner surface is not spread or smoothed with a tool such as a squeegee to achieve uniformity of the silicone oil, it is possible to provide a syringe barrel that has suppressed generation of scratches or adhesion of foreign matter on the barrel inner surface.

In the syringe barrel described above, the silicone oil may have been applied from a vicinity of the opening portion beyond the insertion position to the position closer to the shoulder portion.

With this configuration, the silicone oil has been applied up to a vicinity of the opening portion, making it possible to plug the gasket quickly and reliably up to the insertion position at the time of vacuum plugging.

In the syringe barrel described above, the silicone oil may have been applied up to a vicinity of the shoulder portion.

With this configuration, the silicone oil has been applied to substantially an entire region of the barrel inner surface on which the gasket slides before discharging the drug, making it possible to suppress the sliding resistance of the gasket up to the end.

In the above-described syringe barrel, an average value of the coverage from a vicinity of the shoulder portion to the insertion position may be 10% or less.

This makes it possible to further suppress the sliding resistance of the gasket.

In addition, a prefilled syringe not according to the present invention includes any of the above-described syringe barrels, the gasket, and a drug solution filled in the syringe barrel.

In the above-described prefilled syringe, the drug solution may be a protein preparation.

According to the present invention, there is provided a syringe barrel, and a method for manufacturing a syringe barrel.

### Brief Description of Drawings

FIG. 1 is a perspective view of a syringe including a syringe barrel according to an embodiment of the present invention.
FIG. 2 is a cross-sectional view of the syringe illustrated in FIG. 1.
FIG. 3 is a cross-sectional view of a barrel constituting the syringe illustrated in FIG. 1.
FIG. 4 is a cross-sectional view of a medical package.
FIG. 5A is an illustration of a step of providing a barrel. FIG. 5B is an illustration of a step of applying a silicone oil to a barrel inner surface. FIG. 5C is an illustration of a step of attaching a cap to the barrel.
FIG. 6A is a cross-sectional view of a storage container storing a barrel. FIG. 6B is a cross-sectional view of a sterilization bag storing the storage container.
FIG. 7A is a graph illustrating the silicone oil distribution before sterilization of a sample A1 not filled with water. FIG. 7B is a graph illustrating the silicone oil distribution before sterilization of a sample A2 not filled with water. FIG. 7C is a graph illustrating the silicone oil distribution before sterilization of a sample A3 not filled with water. FIG. 7D is a graph illustrating the silicone oil distribution before sterilization of a sample A4 not filled with water.
FIG. 8A is a graph illustrating the silicone oil distribution after sterilization of the sample A1 not filled with water. FIG. 8B is a graph illustrating the silicone oil distribution after sterilization of the sample A2 not filled with water. FIG. 8C is a graph illustrating the silicone oil distribution after sterilization of the sample A3 not filled with water. FIG. 8D is a graph illustrating the silicone oil distribution after sterilization of the sample A4 not filled with water.
FIG. 9A is a graph illustrating the silicone oil distribution before sterilization of a sample B1 filled with water. FIG. 9B is a graph illustrating the silicone oil distribution before sterilization of a sample B2 filled with water. FIG. 9C is a graph illustrating the silicone oil distribution before sterilization of a sample B3 filled with water. FIG. 9D is a graph illustrating the silicone oil distribution before sterilization of a sample B4 filled with water.
FIG. 10A is a graph illustrating the silicone oil distribution after sterilization of the sample B1 filled with water. FIG. 10B is a graph illustrating the silicone oil distribution after sterilization of the sample B2 filled with water. FIG. 10C is a graph illustrating the silicone oil distribution after sterilization of the sample B3 filled with water. FIG. 10D is a graph illustrating the silicone oil distribution after sterilization of the sample B4 filled with water.

### Description of Embodiments

Preferred embodiments of a syringe barrel, a prefilled syringe and methods for manufacturing these according to the present invention will be described below with reference to the accompanying drawings.

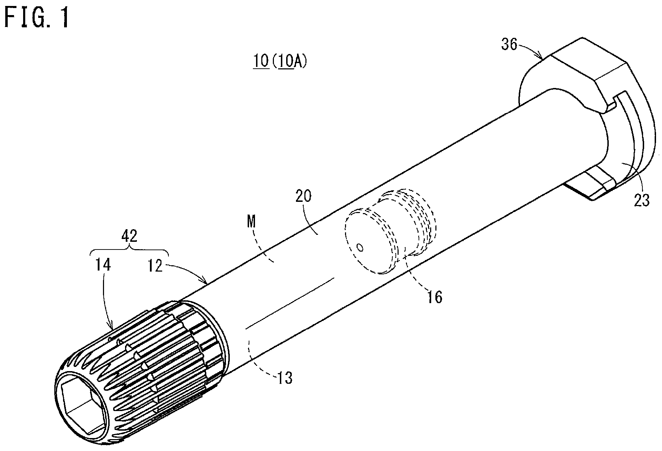

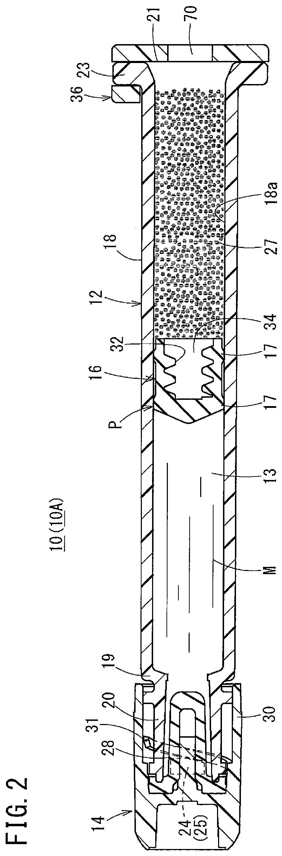

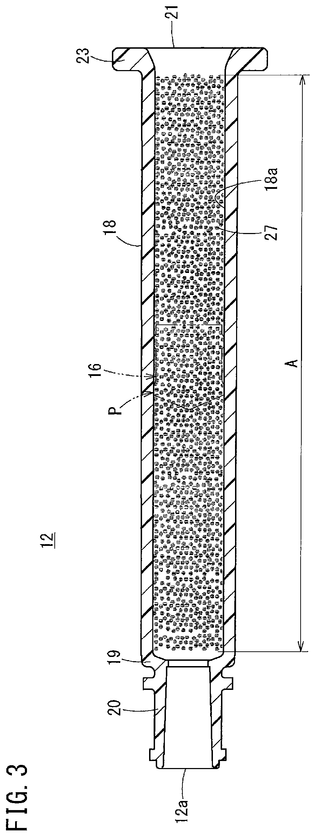

In FIGS. 1 and 2, a syringe 10 includes a syringe barrel 12 (hereinafter simply referred to as a "barrel 12"), a cap 14 for sealing a distal end opening 12a (refer to FIG. 3) of the barrel 12, a gasket 16 inserted into the barrel 12 in a liquid-tight slidable manner, and a drug M filled in a filling chamber 13 formed in the barrel 12. The syringe 10 is configured as a prefilled syringe 10A prefilled with the drug M.

In using the syringe 10, another prefilled syringe having a male luer and filled with a medical liquid (diluting or dissolving liquid) is connected to the syringe 10. Subsequently, in the connected state, the drug M in the syringe 10 is aspirated into the other prefilled syringe filled with the medical liquid to mix the drug M with the medical liquid in the other prefilled syringe to prepare a desired drug solution.

As illustrated in FIGS. 2 and 3, the barrel 12 includes a hollow body portion 18 constituting a main portion of the barrel 12, a shoulder portion 19 reduced in diameter from a distal end of the body portion 18, a hollow nozzle 20 protruding in a distal end direction from the shoulder portion 19, and a proximal end opening portion 21 that opens at a proximal end of the body portion 18.

The body portion 18 is a hollow cylindrical portion in which the gasket 16 is slidably inserted. A flange 23 protruding outward in the radial direction is formed on the outer circumferential portion of the proximal end of the body portion 18.

In order to enhance the slidability of the gasket 16 against the inner surface of the body portion 18 (hereinafter referred to as a "barrel inner surface 18a"), a non-reactive silicone oil 27 having a viscosity of 3000 cps to 30000 cps is applied as a lubricant to the barrel inner surface 18a. Unlike the reactive silicone oil that is crosslinked and cured by heat or the like, the non-reactive silicone oil 27 is not crosslinked and cured even when it is heated. An example of this non-reactive silicone oil 27 is dimethylpolysiloxane. Hereinafter, the non-reactive silicone oil 27 is simply referred to as the "silicone oil 27".

As illustrated in FIG. 3, the silicone oil 27 is applied at least to a portion from an insertion position P, where the gasket 16 is inserted, up to a position closer to the shoulder portion 19 than the insertion position P. Note that the insertion position P is an initial position of the gasket 16 in the syringe 10.

In the present embodiment, the silicone oil 27 is applied to a portion from the vicinity of the proximal end opening portion 21 beyond the insertion position P up to the position closer to the shoulder portion 19, as indicated by an application range with arrow A in FIG. 3. In addition, in the present embodiment, the silicone oil 27 is applied up to the vicinity of the shoulder portion 19. Therefore, in the case of the present embodiment, the silicone oil 27 is applied to substantially an entire region of the barrel inner surface 18a on which the gasket 16 slides before discharging the drug. Note that the vicinity of the proximal end opening portion 21 indicates a region extending in approximately 0 mm to 10 mm from the proximal end opening portion 21 toward the distal end, for example. The vicinity of the shoulder portion 19 indicates a region extending in 0 mm to 10 mm from a boundary portion between the body portion 18 and the shoulder portion 19 (portion where the inner diameter starts to be reduced) toward the proximal end, for example.

As will be described below, the silicone oil 27 provided on the barrel inner surface 18a is first applied to the barrel inner surface 18a and then broadened in a direction of the barrel inner surface 18a and the direction of weight with reduced viscosity due to the heat generated at autoclave sterilization, without being spread or smoothed by a tool such as a squeegee in a state where the barrel 12 is not filled with the drug solution. This uniformizes the silicone oil distribution on the barrel inner surface 18a.

Specifically, a coverage as a ratio of the area of the silicone oil 27 present as droplets to the area of the barrel inner surface 18a is 20% or less in each of sections obtained by dividing a region (region indicated by arrow A in FIG. 3) on the barrel inner surface 18a to which the silicone oil 27 is applied, at a predetermined width in an axial direction. Each of sections is an annular section. In addition, in the present embodiment, an average value of the coverage at a portion from the vicinity of the shoulder portion 19 up to the insertion position P is 10% or less.

An exemplary method of measuring the coverage of the silicone oil 27 includes a scheme of detecting an edge of a transparent fine particle by forming bright and dark light fringes (zebra pattern) on an object to quantify an application state (hereinafter referred to as a " zebra pattern measurement scheme ") . Measurement of the coverage using the zebra pattern measurement scheme is specifically implemented by emitting light from a light source (for example, a red light source) via a slit plate having a large number of slits formed at predetermined intervals so as to form bright and dark fringes in which the bright and dark portions alternate in the axial direction with respect to the body portion 18 of the barrel 12. Next, the barrel is photographed by receiving transmitted light transmitted through the body portion 18 of the barrel 12, so as to detect a ring-shaped edge from the shading in a photographed image. This photographing and edge detection is performed on the entire circumference of the barrel 12 by rotating the barrel 12 by a predetermined angle. Subsequently, the droplet of the silicone oil 27 is specified from the edge, and a proportion (%) of the total area of the droplet to the image area is defined as the coverage. More specifically, the region on the barrel inner surface 18a to which the silicone oil 27 is applied is divided into sections (annular bands) having a predetermined width in the axial direction, so as to calculate the coverage for each of the sections. In this case, the predetermined width of the section is set to 1 mm to 3 mm, for example.

In the case of the barrel 12, the silicone oil 27 applied to the barrel inner surface 18a is not spread or smoothed after being sprayed onto the barrel inner surface 18a, and thus, a droplet structure of the silicone oil 27 exists on the barrel inner surface 18a. The droplet structure of the silicone oil 27 is a raised structure or an uneven structure formed by the droplet of the applied silicone oil 27 not spreading out completely even by heating. In contrast, the droplet structure of the silicone oil 27 would not exist on the barrel inner surface 18a in a case where the silicone oil 27 is spread and smoothed with a tool such as a squeegee after the silicone oil 27 is applied to the barrel inner surface 18a as in the above-described conventional technique.

The nozzle 20 is reduced in diameter with respect to the body portion 18 from the distal end portion of the body portion 18 and protrudes in the distal end direction. The nozzle 20 includes a female luer to which the male luer can be inserted and connected, and has a tapered inner surface with an inner diameter increasing toward the distal end direction.

In FIG. 2, the outer circumferential portion of the distal end of the nozzle 20 includes a fixing portion 24 for detachably fixing the cap 14. In the present embodiment, the fixing portion 24 is constituted by two engaging projections 25 protruding in directions opposite to each other about an axis of the barrel 12 as a reference and capable of being screwed with the cap 14.

Examples of the constituent material of the barrel 12 include various resins such as polypropylene, polyethylene, polystyrene, polyamide, polycarbonate, polyvinyl chloride, poly-(4-methylpentene-1), acrylic resin, acrylonitrile-butadiene-styrene copolymer, polyester such as polyethylene terephthalate, cyclic olefin polymer, and cyclic olefin copolymer. Among them, resins such as polypropylene, cyclic olefin polymer and cyclic olefin copolymer are preferable because of their excellent moldability and heat resistance. Moreover, as will be described below, the silicone oil 27 tends to spread out with higher uniformity by heat during thermal treatment (autoclave sterilization) in the case of the cyclic olefin polymer than the polypropylene or the like, and thus, it is more preferable that the barrel 12 be formed of cyclic olefin polymer.

The cap 14 includes a sealing member 28 formed of an elastic member for sealing the distal end opening 12a of the barrel 12, and a tubular main body portion 30 supporting the sealing member 28. The inner circumferential portion of the main body portion 30 includes a female screw 31 to be screwed into the fixing portion 24 (engaging projection 25) on the nozzle 20. In a pre-use state in which the cap 14 is attached to the nozzle 20, the distal end opening 12a is sealed liquid-tight by the cap 14 so as to suppress leakage of the drug M from the distal end opening 12a.

The outer circumferential portion of the gasket 16 includes a plurality of ring-shaped sealing projections 17 formed at intervals in the axial direction. With the gasket 16 inserted in the barrel 12, the sealing projection 17 comes into close contact with the barrel inner surface 18a. This enables the gasket 16 to slide within the barrel 12 in a liquid-tight manner in the axial direction.

The gasket 16 includes a fitting recess 34 opening to the proximal end side and having a female screw 32 formed in the inner circumferential portion. The fitting recess 34 can be screwed with the distal end portion of a pusher (not illustrated) as necessary.

Examples of the constituent material of the gasket 16 include various rubber materials such as natural rubber, butyl rubber, isoprene rubber, butadiene rubber, styrene-butadiene rubber, and silicone rubber, or various types of thermoplastic elastomer such as polyurethane type, polyester type, polyamide type, olefin type, and styrene type, or a mixture of these.

The drug M may be any of a powdered drug, a lyophilized drug, a solid drug, a liquid drug, or the like. Examples of the drug include protein preparations, antitumor agents, vitamins (multivitamin), various amino acids, antithrombotics such as heparin, insulin, antibiotics, analgesics, cardiotonics, intravenous anesthetics, medical narcotics, antiparkinson agents, ulcer therapeutic agents, adrenocortical hormone agents, and arrhythmia agents.

Note that the syringe 10 includes, at the proximal end portion (flange 23) of the barrel 12, a detachable gasket stopper 36 that prevents the gasket 16 from dropping from the barrel 12 in the proximal end direction.

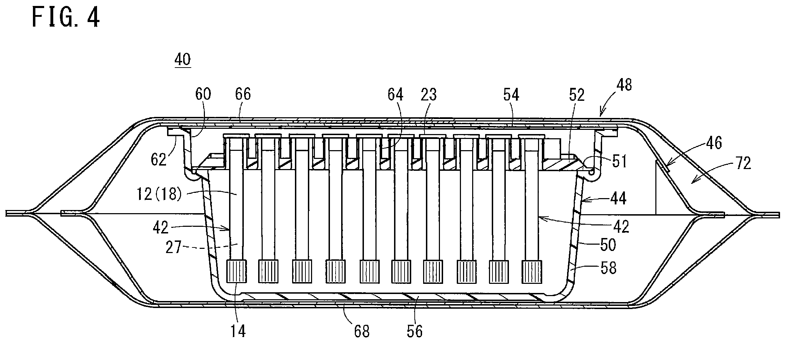

The medical package 40 illustrated in FIG. 4 includes one or more syringe assemblies 42, a storage container 44 for storing the syringe assembly 42, a sterilization bag 46 for storing the storage container 44, and outer wrap 48 for storing the sterilization bag 46. The syringe assembly 42 is an assembly constituting a portion of the syringe 10 (prefilled syringe 10A) described above. Specifically, the syringe assembly 42 includes the barrel 12 having the silicone oil 27 being applied to the barrel inner surface 18a, and the cap 14 attached to the distal end portion of the barrel 12. The barrel 12 of the syringe assembly 42 is not filled with the drug M (refer to FIG. 2).

The storage container 44 includes a container main body 50 (tub), a holding member 52 (nest), and a sheet material 54. The container main body 50 is formed in a box shape including a bottom portion 56 forming a bottom wall, a side portion 58 forming a circumferential wall, and a flange portion 62 surrounding an opening portion 60 formed at an upper end portion of the side portion 58. A sheet material 54 is releasably fixed (bonded) to an upper surface of the flange portion 62.

The holding member 52 is mounted on a step 51 formed in the container main body 50, so as to hold a plurality of syringe assemblies 42 flush with each other. The holding member 52 includes a plurality of hollow cylindrical protrusion holders 64. The flange 23 provided at the proximal end of the barrel 12 is caught on the upper end of the protrusion holder 64 so as to hold the syringe assembly 42 in a substantially vertically suspended state.

The sheet material 54 is a lid member to seal the opening portion 60 of the container main body 50, and is formed of a material having gas permeability and bacteria impermeability. Accordingly, the sheet material 54 is permeable to water vapor used as a sterilizing gas at the time of autoclave sterilization (high-pressure steam sterilization) in the step of manufacturing the medical package 40. Examples of the constituent material of the sheet material 54 include a plastic nonwoven fabric and a plastic porous film. An example of the plastic nonwoven fabric is nonwoven polyolefin.

The sterilization bag 46 is a bag having, at least partially, gas permeability and bacteria impermeability. In the present embodiment, the sterilization bag 46 includes a first sheet 66 having gas permeability and bacteria impermeability, andasecond sheet 68 formed of a material having gas impermeability and bacteria impermeability (for example, polyethylene). Peripheral edges of the first sheet 66 and the second sheet 68 are fusion bonded to each other.

The outer wrap 48 is a bag formed of a material having gas impermeability and bacteria impermeability (for example, polyethylene), being formed to be larger than the sterilization bag 46 so as to be able to store the sterilization bag 46 storing the storage container 44. As will be described below, after the sterilization bag 46 storing the storage container 44 is wrapped in the outer wrap 48 in the manufacturing step, a mouth portion of the outer wrap 48 is sealed by a sealing apparatus to achieve outer wrap of the sterilization bag 46.

The medical package 40 thus configured is shipped in a box and unsealed by a pharmaceutical manufacturer, for example, and the syringe assembly 42 is taken out. Subsequently, the drug M is filled in the barrel 12 of the syringe assembly 42, then, the gasket 16 is inserted into the barrel 12, so as to complete the prefilled syringe 10A illustrated in FIG. 1. The method for manufacturing the prefilled syringe 10A according to the present embodiment includes a preparation step of preparing the barrel 12, a filling step of filling the prepared liquid barrel 12 with a drug solution, and a plugging step of plugging the gasket 16 into the barrel 12 filled with the drug solution.

The medical package 40 configured as described above can be manufactured by a manufacturing method including, for example, the following steps. In the present embodiment, the method for manufacturing the barrel 12 having the silicone oil 27 uniformly applied to the barrel inner surface 18a includes a providing step, an application step, and a heating step as described below, the method constituting a portion of the manufacturing method of the medical package 40.

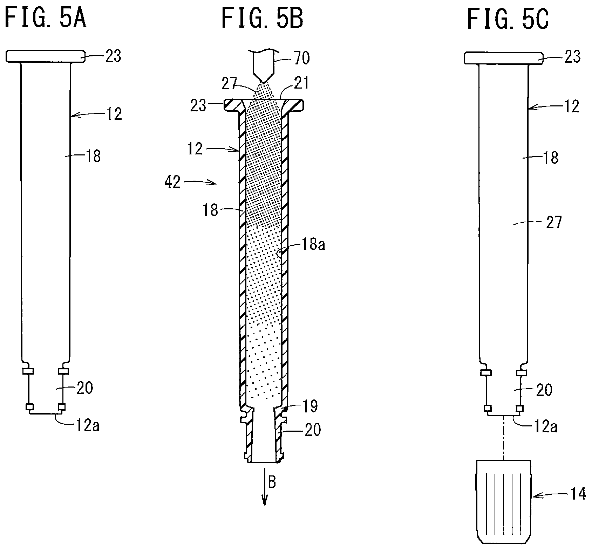

As illustrated in FIG. 5A, the barrel 12 is provided (providing step). The barrel 12 can be manufactured by an injection molding method, for example.

Next, as illustrated in FIG. 5B, the silicone oil 27 having a viscosity of 3000 cps to 30000 cps is applied to the barrel inner surface 18a by spraying with a coating amount rate of 0.02 mg/cm² to 0.2 mg/cm² (application step) . The coating amount rate is a value obtained by dividing the weight of the silicone oil 27 dispensed from a spray nozzle 70 by the area of the barrel inner surface 18a.

Specifically, in the application step, the barrel 12 is held with the proximal end opening portion 21 facing upward (with the cap 14 downward), and the spray nozzle 70 is arranged to face the proximal end opening portion 21. Subsequently, the silicone oil 27 is dispensed from the spray nozzle 70 in a form of mist and sprayed onto the barrel inner surface 18a. That is, the silicone oil 27 is sprayed from the proximal end opening portion 21 of the barrel 12 toward the nozzle 20 side onto the barrel inner surface 18a. This operation applies the silicone oil 27 to the barrel inner surface 18a. In the present embodiment, the silicone oil 27 is applied to a portion from the vicinity of the proximal end opening portion 21 of the barrel 12 up to the vicinity of the shoulder portion 19.

In addition, as illustrated in FIG. 5B, in the present embodiment, air B is aspirated from the nozzle 20 side when the silicone oil 27 is applied by spraying. With a method of spraying the silicone oil 27 while aspirating the air B from the nozzle 20 side in this manner, the silicone oil 27 can be reliably applied up to the vicinity of the shoulder portion 19. Moreover, it is possible to reduce unevenness of spraying and apply the silicone oil 27 with higher uniformity.

Next, as illustrated in FIG. 5C, the cap 14 is provided and then, the cap 14 is attached to the nozzle 20 of the barrel 12 with the silicone oil 27 applied, so as to seal the distal end opening 12a of the barrel 12.

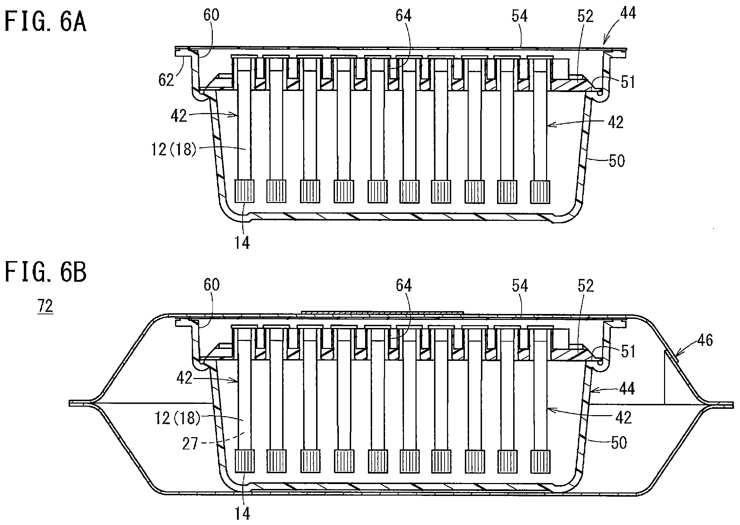

Next, a step of obtaining the storage container 44 illustrated in FIG. 6A is performed. At first for this step, one or more syringe assemblies 42 obtained as described above are stored in the container main body 50. Specifically, the syringe assembly 42 is inserted into each of the protrusion holders 64 of the holding member 52 with the distal end side facing downward. Next, the plurality of syringe assemblies 42 is suspended by the holding member 52, and thereafter, the holding member 52 is mounted on the inside of the container main body 50.

Next, the opening portion 60 of the container main body 50 is sealed with the sheet material 54. Specifically, the sheet material 54 is releasably fixed to the flange portion 62 of the container main body 50, so as to seal the opening portion 60 of the container main body 50. In this case, it is allowable to use fixation with an adhesive in addition to thermal fusion bonding, as a fixing means. The opening portion 60 of the container main body 50 storing the syringe assembly 42 is sealed with the sheet material 54. With this processing, the storage container 44 storing the syringe assembly 42 not filled with the drug M is obtained as illustrated in FIG. 6A.

Next, a step of obtaining a package 72 illustrated in FIG. 6B is performed. For this, first, the storage container 44 (container main body 50 in which the syringe assembly 42 is stored and the opening portion 60 is sealed with the sheet material 54) is packaged by the sterilization bag 46. Specifically, the storage container 44 is stored in the sterilization bag 46 with its mouth portion open, and thereafter, the mouth portion of the sterilization bag 46 is sealed. This processing achieves acquisition of the package 72, in which the opening portion 60 of the container main body 50 is sealed with the sheet material 54, the syringe assembly 42 is stored in the storage container 44, and the storage container 44 is stored in the sterilization bag 46.

Next, the package 72 undergoes sterilization treatment. In the present embodiment, autoclave sterilization is performed as the sterilization treatment. In autoclave sterilization, water vapor as a sterilizing gas permeates through the sterilization bag 46, so as to allow the syringe assembly 42 in the storage container 44 to be sterilized. In addition, the silicone oil 27 applied to the barrel inner surface 18a has a reduced viscosity due to the heat generated at autoclave sterilization. That is, in the present embodiment, the autoclave sterilization also serves as a heating step of heating the silicone oil 27 applied to the barrel inner surface 18a to reduce its viscosity in a state where the barrel 12 is not filled with liquid.

At this time, since the drug solution is not filled in the barrel 12, the silicone oil 27 with reduced viscosity is greatly influenced by surface tension and gravity. This allows the silicone oil 27 to spread out in a direction of a wall surface (barrel inner surface 18a) and the direction of gravity, leading to uniformization of the silicone oil distribution on the barrel inner surface 18a.

After completion of sterilization of the package 72, the package 72 is stored in the outer wrap 48 with its mouth portion open, and then, the mouth portion of the outer wrap 48 is sealed, so as to store the package 72 in the outer wrap 48. This achieves acquisition of the medical package 40 illustrated in FIG. 4.

Note that while the above example is a case where autoclave sterilization is performed as a sterilization treatment, and the silicone oil 27 applied to the barrel inner surface 18a is heated by the heat generated at autoclave sterilization so as to reduce the viscosity, the heating of the silicone oil 27 may be performed by thermal treatment other than autoclave sterilization. In this case, the sterilization treatment may be ethylene oxide sterilization, electron beam sterilization, or the like.

As described above, according to the present embodiment, the silicone oil 27 applied to the barrel inner surface 18a is heated to reduce the viscosity, so as to allow the silicone oil 27 to spread out in a direction of the barrel inner surface 18a and the direction of gravity. This uniformizes the silicone oil distribution on the barrel inner surface 18a, making it possible to suppress the sliding resistance of the gasket 16 inserted into the barrel 12.

In this case, excessively high viscosity of the silicone oil 27 applied to the barrel inner surface 18a would lead to difficulty in reducing the viscosity even by the heat generated at heat treatment and in achieving uniformity in the silicone oil 27. Conversely, excessively low viscosity of the silicone oil 27 would allow the silicone oil 27 to flow excessively in the direction of gravity during the heat treatment. In the present embodiment, the silicone oil 27 having a viscosity of 3000 cps to 30000 cps is applied to suitably reduce the viscosity of the silicone oil 27 at the time of heat treatment, so as to achieve effective uniformization of the silicone oil distribution.

Furthermore, in the present embodiment, the silicone oil 27 is applied to the barrel inner surface 18a with a coating amount rate of 0.02 mg/cm² to 0.2 mg/cm², making it possible to allow the silicone oil 27 to uniformly spread over the barrel inner surface 18a without excessively applying the silicone oil 27.

In addition, since it is possible to avoid excessive application of the silicone oil 27 and the silicone oil 27 is not formed as large particles, outflow of the silicone oil 27 into the drug solution in the prefilled syringe 10A is expected to be suppressed. Since the silicone oil 27 flowing out into the drug solution would cause aggregation of the protein preparation, it is particularly preferable to suppress outflow of the silicone oil 27 into the drug solution in a case of filling the protein preparation. Moreover, since the particles of the silicone oil 27 are greatly reduced, it is possible to prevent bubbles generated at the time of filling the drug in the filling chamber 13 of the barrel 12 from being caught on the barrel inner surface 18a. This makes it possible to suppress false detection of foreign matter due to bubbles at the time of foreign matter inspection of the prefilled syringe 10A.

In addition, since the silicone oil 27 applied to the barrel inner surface 18a is not spread or smoothed with a tool such as a squeegee to achieve uniformity in the silicone oil distribution, it is possible to obtain the barrel 12 on which the silicone oil 27 is uniformly applied without generation of scratches or adhesion of foreign matter on the barrel inner surface 18a.

With the present embodiment, the silicone oil 27 is heated at the time of autoclave sterilization for the barrel 12, making it possible to omit providing a dedicated step for heating the silicone oil 27. Therefore, it is possible to efficiently manufacture the barrel 12 with the silicone oil 27 applied with high uniformity. In addition, autoclave sterilization can suppress degradation of the barrel 12 as compared with other sterilization treatments such as electron beam sterilization, making it possible to suppress adverse effects on the drug solution filled in the barrel 12.

In the present embodiment, the heating step is performed in a state where the barrel 12 faces up and down directions. This allows the silicone oil 27 to spread out in a barrel 12 axial direction due to gravity at the time of heating, leading to higher uniformity in the silicone oil distribution.

When the silicone oil 27 is applied to the barrel inner surface 18a by spraying, the coating amount rate of the silicone oil 27 is increased toward the side closer to the spray nozzle 70 (refer to FIG. 5B), and the mass of the silicone oil 27 is likely to be formed. In the present embodiment, the application step is performed such that the silicone oil 27 is sprayed from one end side (proximal end opening portion 21 side) of the barrel 12 toward the other end side (nozzle 20 side) to allow the silicone oil 27 to be applied to the barrel inner surface 18a, while the heating step is performed with the other end side facing downward. With this method, heating is performed with the side opposite to the spraying side facing downward, allowing the mass of the silicone oil 27 to spread out from one end side of the barrel 12 toward the other end side due to gravity. This makes it possible to effectively achieve higher uniformity in the silicone oil distribution.

In the present embodiment, the silicone oil 27 is applied to a portion from the vicinity of the proximal end opening portion 21 beyond the insertion position P up to the position closer to the shoulder portion 19. With this configuration, the silicone oil 27 is applied up to the vicinity of the proximal end opening portion 21, making it possible to plug the gasket 16 quickly and reliably up to the insertion position P at the time of vacuum plugging.

In particular, in the present embodiment, the silicone oil 27 is applied up to the vicinity of the shoulder portion 19. With this configuration, the silicone oil 27 is applied to substantially an entire region of the barrel inner surface 18a on which the gasket 16 slides before discharging the drug, making it possible to suppress the sliding resistance of the gasket 16 up to the end.

In addition, in the present embodiment, the average value of the coverage at a portion from the vicinity of the shoulder portion 19 up to the insertion position P is 10% or less, making it possible to further suppress the sliding resistance of the gasket 16.

In order to confirm the effects of the present invention described above, the following test was performed.

In the test, 0.6 mg of silicone oil was dispensed from a spray nozzle in a form of mist to be applied to the barrel inner surface with the spray nozzle arranged to face the proximal end opening portion of the barrel having a capacity of 2.25 mL. The silicone oil used was DOW CORNING (R) 360 MEDICAL FLUID, 12500 CST (manufactured by Dow Corning Corporation, viscosity: 12500) .

Ten barrels with the silicone oil applied to the inner surface in this manner were prepared. Among the ten barrels , five barrels (samples A1 to A5) are each provided with a cap attached and without water filled therein, while the other five barrels (samples B1 to B5) are each provided with a cap attached and with water filled. Thereafter, the gasket was inserted into the barrels and sealed.

Subsequently these samples A1 to A5 and B1 to B5 underwent autoclave sterilization (121°C, 20 minutes) and are then dried. Autoclave sterilization was performed with the barrel nozzle facing downward and the barrel kept in a substantially vertical posture. For samples B1 to B5 filled with water, the water in each of the barrels was removed after sterilization and drying.

For each of the samples, silicone oil distribution on the barrel inner surface was measured by the above-described zebra pattern measurement scheme and then evaluated. The measurement instrument used was ZS-S40 Bench Top Silicone Detection System (manufactured by ZEBRASCI). In the measurement, photographing was performed six times while the barrel was rotated by 63° to measure the entire barrel, and then, an overlapping portion (corresponding to 18°) was removed. In addition, the silicone oil application region on the barrel inner surface was divided into 35 sections in the axial direction, and the coverage was measured for each of the sections. From the results obtained, it was investigated how the silicone oil distribution in the barrel changes before and after sterilization.

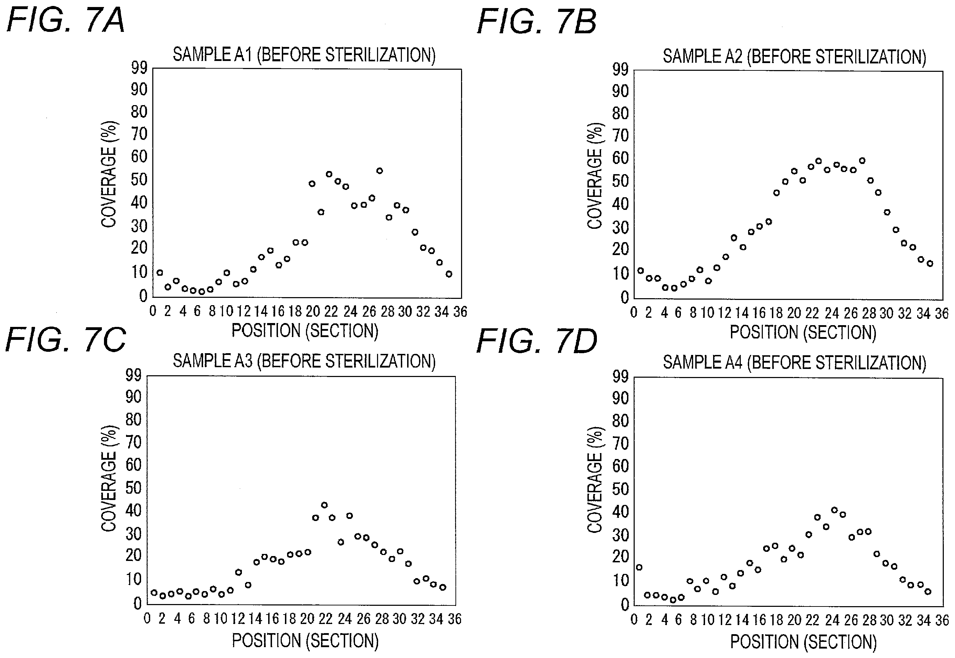

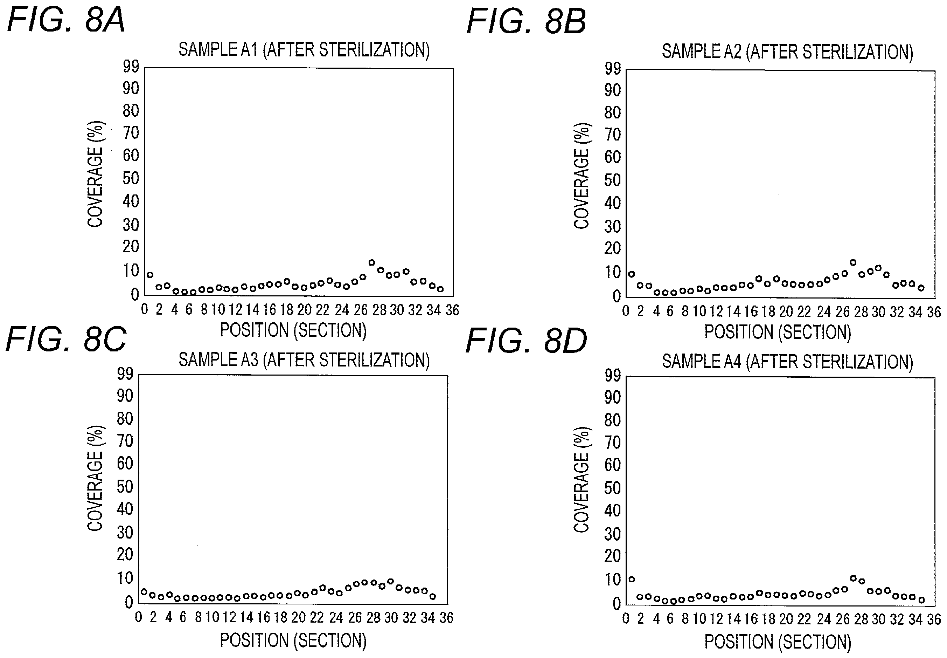

FIGS. 7A to 7D are graphs illustrating silicone oil distribution before autoclave sterilization of samples A1 to A4 that were not filled with water. FIGS. 8A to 8D are graphs illustrating silicone oil distribution after autoclave sterilization of samples A1 to A4 that were not filled with water. In each of the graphs, the horizontal axis indicates the section number corresponding to the position of the barrel in the axial direction (left side is the shoulder portion side and the right side is the proximal end opening portion side), while the vertical axis indicates the coverage in each of the positions (sections) . From the graphs of FIGS. 7A to 7D, it is observed that the coverage is high on the proximal end side of the barrel close to the spray nozzle (oil dispense portion) before autoclave sterilization, and that the silicone oil distribution is not uniform.

In contrast, it is observed from the graphs of FIGS. 8A to 8D that, after autoclave sterilization of the samples A1 to A4 that were not filled with water, the coverage was 20% or less even at maximum, and that the silicone oil distribution is uniform. In addition, the average coverage after autoclave sterilization was 10% or less in each of the samples. Note that, although not illustrated here, it was observed from the images before and after sterilization of each of sample A1 to A4 that mass of silicone oil has spread out to the cap side by sterilization.

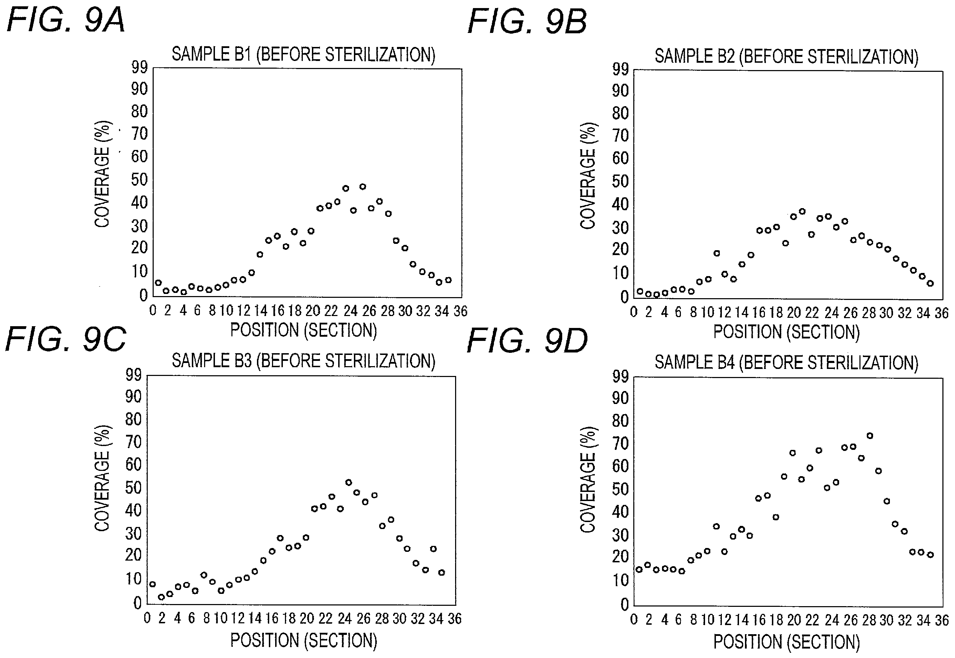

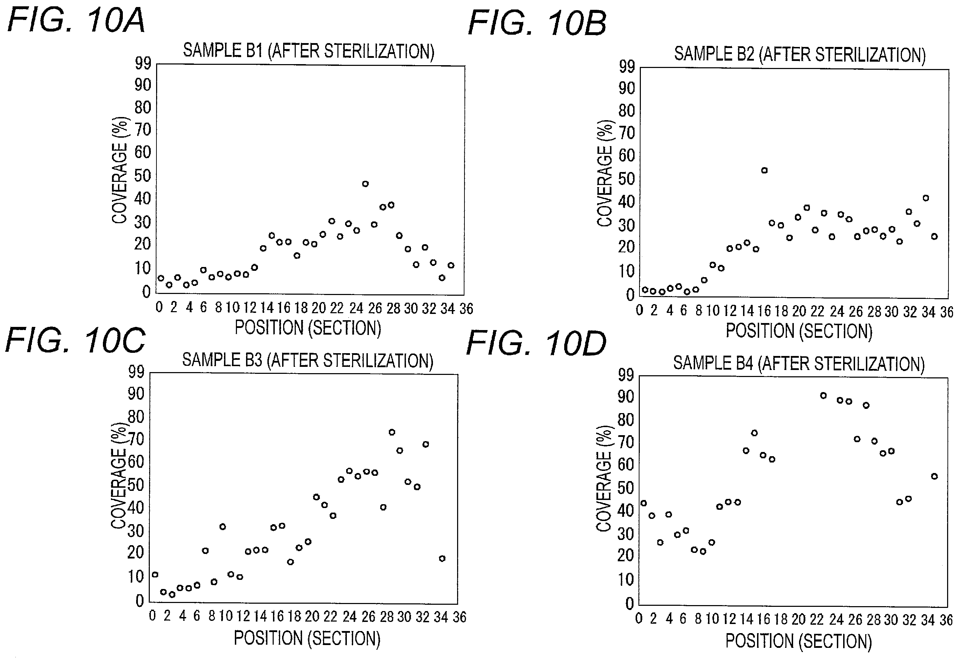

FIGS. 9A to 9D are graphs illustrating silicone oil distribution before autoclave sterilization of samples B1 to B4 that were filled with water. FIGS. 10A to 10D are graphs illustrating silicone oil distribution after autoclave sterilization of samples B1 to B4 that were filled with water. From these graphs, it was observed that sterilization increases the coverage. In addition, although not illustrated here, the mass of silicone oil did not seem to have spread out before and after sterilization on the basis of an image before and after sterilization of each of samples B1 to B4. This coverage increase by sterilization is presumed to be caused by aggregation of the silicone oil during sterilization due to water pressure. Note that the excessively high coverage in a region on the proximal end opening portion side in the graph after sterilization of the sample filled with water (particularly, FIGS. 10B to 10D) is presumed to be caused by extrusion of the silicone oil on the proximal end opening portion side at the time of insertion of the gasket into the barrel. For this reason, this region was excluded from the evaluation target.

The difference in silicone oil distribution due to the presence or absence of water filling as described above can be considered and explained as follows. That is, in the barrels (samples A1 to A4) not filled with water, the viscosity of the silicone oil is reduced by heat generated at sterilization, and the oil is greatly influenced by surface tension and gravity. As a result, the silicone oil spreads out in the direction of the wall surface and the direction of gravity. In contrast, in the barrels filled with water (samples B1 to B4), the silicone oil is greatly influenced by water pressure at this time although the viscosity of the silicone oil is reduced by the heat during sterilization. As a result, the silicone oil is aggregated.

From the above test results, it was observed that the silicone oil applied to the barrel not filled with water achieves higher uniformity by heat treatment. In contrast, it was observed that the silicone oil applied to the barrel filled with water hardly moved or even aggregated after heat treatment.

As described above, in a case where heat treatment (for example, autoclave sterilization) is performed without the drug solution filled, the distribution of the silicone oil 27 applied to the barrel inner surface 18a achieves higher uniformity as compared with the case where the heat treatment is performed with the drug solution filled. This characteristic contributes to enhancement of the slidability of the gasket 16 inserted into the barrel 12.

Note that each of the above-described embodiments is a case where the barrel 12 is configured as a barrel for the prefilled syringe 10A prefilled with the drug M, the present invention can also be applied to a syringe barrel not prefilled with the drug M.

In addition, the nozzle 20 is not particularly limited as long as it has a shape capable of discharging the drug M in the barrel 12, and thus, may have a shape of a male luer that can be inserted and connected to a female luer, a shape having a lock adapter around the male luer, and a shape in which an injection needle is directly fixed inside the nozzle. Alternatively, the barrel 12 may have a cylindrical shape having no nozzle and having a distal end opening portion at the other end opposite to the proximal end opening portion 21. In this case, a sealing member for sealing the distal end opening portion would be attached to the other end (distal end) of the barrel 12 and used together with a needle hub or the like having a double-ended needle.

The present invention is not limited to the above-described embodiment, and various modifications are possible without departing from the scope of the invention as defined in the appended claims.

## Claims

1. A method for manufacturing a syringe barrel (12), comprising:
a providing step of providing the syringe barrel (12) including a hollow body portion (18) and being configured that a gasket (16) sliding on an inner surface (18a) of the body portion (18) is inserted therein;
an application step of applying a non-reactive silicone oil (27) having a viscosity of 3000 cps to 30000 cps to the inner surface (18a) by spraying with a coating amount rate of 0.02 mg/cm² to 0.2 mg/cm²; and
a heating step of heating the silicone oil (27) applied to the inner surface (18a) without spreading or smoothing, and being heated by performing autoclave sterilization on the syringe barrel (12) to reduce the viscosity so as to allow the silicone oil to spread out in a direction of the inner surface (18a) of the body portion (18) and the directions of gravity, the silicone oil (27) applied to the inner surface (18a) in a state where a liquid is not filled in the syringe barrel (12).

2. The method for manufacturing the syringe barrel (12) according to claim 1, further comprising a step of obtaining a package (72) by storing the syringe barrel (12) in the sterilization bag (46).

3. The method for manufacturing the syringe barrel (12) according to claim 1 or 2,
wherein the heating step is performed in a state where an axis of the syringe barrel (12) is substantially vertical.

4. The method for manufacturing the syringe barrel (12) according to claim 3,
wherein, in the application step, the silicone oil (27) is applied to the inner surface (18a) by spraying the silicone oil (27) from one end side toward the other end side of the syringe barrel (12), and
the heating step is performed in a state where the other end side faces downward.

5. The method for manufacturing the syringe barrel (12) according to any one of claims 1 to 4,
wherein, in the application step, the silicone oil (27) is sprayed from one end side toward the other end side of the syringe barrel (12), while air is aspirated within the syringe barrel (12) from the other end side.

6. The method for manufacturing the syringe barrel (12) according to claim 5,
wherein the syringe barrel (12) includes: an opening portion (21) for inserting the gasket (16) at the one end side; and a nozzle (20) having an inner diameter smaller than the inner diameter of the body portion (18) at the other end side.

7. A method for manufacturing a prefilled syringe, comprising:
a preparation step of preparing the syringe barrel (12) manufactured by the method for manufacturing the syringe barrel (12) according to any one of claims 1 to 6;
a filling step of filling the prepared syringe barrel (12) with a drug solution; and
a plugging step of plugging the gasket (16) into the syringe barrel (12) filled with the drug solution.

8. The method for manufacturing a prefilled syringe according to claim 7,
wherein the drug solution is a protein preparation.

9. A syringe barrel (12) comprising: a hollow body portion (18); a shoulder portion (19) reduced in diameter from a distal end of the body portion (18); a nozzle (20) protruding in a distal end direction from the shoulder portion (19); and an opening portion (21) that opens at a proximal end of the body portion (18), the syringe barrel (12) being configured that a gasket (16) sliding on an inner surface (18a) of the body portion (18) is inserted therein,
wherein a non-reactive silicone oil (27) having a viscosity of 3000 cps to 30000 cps has been applied on the inner surface at least from an insertion position (P) configured for the gasket (16) being inserted thereat, to a position closer to the shoulder portion (19) than the insertion position (P), and
a coverage as a ratio of the area of the silicone oil (27) present as droplets to the area of the inner surface (18a) is 20% or less at each of sections where a region applied with the silicone oil (27) in the inner surface (18a) is partitioned with a predetermined width in the axial direction, and a droplet structure of the silicone oil (27) is present on the inner surface (18a).

10. The syringe barrel (12) according to claim 9,
wherein the silicone oil (27) has been applied from a vicinity of the opening portion (21) beyond the insertion position to the position closer to the shoulder portion (19).

11. The syringe barrel (12) according to claim 10,
wherein the silicone oil (27) has been applied up to a vicinity of the shoulder portion (19).

12. The syringe barrel (12) according to any one of claims 9 to 11,
wherein an average value of the coverage from a vicinity of the shoulder portion (19) to the insertion position is 10% or less.

## Patentansprüche

1. Verfahren zur Herstellung eines Spritzenzylinders (12), umfassend:
einen Bereitstellungsschritt zum Bereitstellen des Spritzenzylinders (12), der einen hohlen Körperabschnitt (18) umfasst und so konfiguriert ist, dass eine Dichtung (16), die auf einer Innenfläche (18a) des Körperabschnitts (18) gleitet, darin eingesetzt ist;
einen Auftragungsschritt zum Auftragen eines nicht reaktiven Silikonöls (27), welches eine Viskosität von 3.000 cP bis 30.000 cP aufweist, auf die Innenfläche (18a) durch Sprühen mit einem Beschichtungsmengen-Verhältnis von 0,02 mg/cm² bis 0,2 mg/cm²; und
einen Erhitzungsschritt des Erhitzens von dem Silikonöl (27), das auf die Innenfläche (18a) ohne Verteilen oder Glätten aufgetragen wird, und welches erhitzt wird über die Durchführung einer Sterilisation von dem Spritzenzylinder (12) in einem Autoklav, um die Viskosität zu verringern, sodass es dem Silikonöl gestattet ist, sich in einer Richtung der Innenfläche (18a) des Körperabschnitts (18) und in den Richtungen der Schwerkraft auszubreiten, wobei das Silikonöl (27) auf die Innenfläche (18a) in einem Zustand aufgetragen wird, in dem keine Flüssigkeit in den Spritzenzylinder (12) eingefüllt ist.

2. Verfahren zur Herstellung des Spritzenzylinders (12) nach Anspruch 1,
ferner umfassend einen Schritt des Erhaltens einer Verpackung (72) durch das Aufbewahren des Spritzenzylinders (12) in dem Sterilisationsbeutel (46).

3. Verfahren zur Herstellung des Spritzenzylinders (12) nach Anspruch 1 oder 2,
wobei der Erhitzungsschritt in einem Zustand durchgeführt wird, in dem eine Achse des Spritzenzylinders (12) im Wesentlichen senkrecht ist.

4. Verfahren zur Herstellung des Spritzenzylinders (12) nach Anspruch 3,
wobei, in dem Auftragungsschritt, das Silikonöl (27) auf die Innenfläche (18a) aufgetragen wird, indem das Silikonöl (27) von einer Endseite in Richtung zu der anderen Endseite des Spritzenzylinders (12) gesprüht wird, und
der Erhitzungsschritt in einem Zustand durchgeführt wird, in welchem die andere Endseite nach unten zeigt.

5. Verfahren zur Herstellung eines Spritzenzylinders (12) nach einem der Ansprüche 1 bis 4,
wobei, in dem Auftragungsschritt, das Silikonöl (27) von einer Endseite in Richtung zu der anderen Endseite des Spritzenzylinders (12) gesprüht wird, während Luft innerhalb des Spritzenzylinders (12) von der anderen Endseite her angesaugt wird.

6. Verfahren zur Herstellung des Spritzenzylinders (12) nach Anspruch 5,
wobei der Spritzenzylinder (12) umfasst: einen Öffnungsabschnitt (21) zum Einführen der Dichtung (16) an der einen Endseite; und eine Düse (20), die einen Innendurchmesser aufweist, der kleiner ist als der Innendurchmesser des Körperabschnitts (18) an der anderen Endseite.

7. Verfahren zur Herstellung einer vorgefüllten Spritze, umfassend:
einen Herstellungsschritt zur Herstellung des Spritzenzylinders (12), der durch das Verfahren zur Herstellung des Spritzenzylinders (12) nach einem der Ansprüche 1 bis 6 hergestellt wurde;
einen Befüllungsschritt zum Befüllen des hergestellten Spritzenzylinders (12) mit einer Arzneimittellösung; und
einen Stopfschritt zum Stopfen der Dichtung (16) in den Spritzenzylinder (12), der mit der Arzneimittellösung befüllt ist.

8. Verfahren zur Herstellung einer vorgefüllten Spritze nach Anspruch 7,
wobei die Arzneimittellösung eine Proteinzubereitung ist.

9. Spritzenzylinder (12), umfassend: einen hohlen Körperabschnitt (18); einen Schulterabschnitt (19), dessen Durchmesser von einem distalen Ende des Körperabschnitts (18) verringert ist; eine Düse (20), die in einer Richtung zum distalen Ende hin aus dem Schulterabschnitt (19) herausragt; und einen Öffnungsabschnitt (21), der sich an einem proximalen Ende des Körperabschnitts (18) öffnet, wobei der Spritzenzylinder (12) so konfiguriert ist, dass eine auf einer Innenfläche (18a) des Körperabschnitts (18) gleitende Dichtung (16) darin eingesetzt ist,
wobei ein nicht reaktives Silikonöl (27), das eine Viskosität von 3.000 cP bis 30.000 cP aufweist, auf die Innenfläche aufgetragen wurde, zumindest von einer Einführungsposition (P), die für das Einführen der Dichtung (16) daselbst konfiguriert ist, bis hin zu einer Position, die näher an dem Schulterabschnitt (19) liegt als die Einführungsposition (P), und
eine Bedeckung als ein Verhältnis der Fläche des Silikonöls (27), das als Tröpfchen vorhanden ist, zu der Fläche der Innenfläche (18a) an jedem der Teilabschnitte, an dem ein mit dem Silikonöl (27) beschichteter Bereich in der Innenfläche (18a) mit einer vorbestimmten Breite in der axialen Richtung unterteilt ist, 20 % oder weniger beträgt und eine Tröpfchenstruktur des Silikonöls (27) auf der Innenfläche (18a) vorhanden ist.

10. Spritzenzylinder (12) nach Anspruch 9,
wobei das Silikonöl (27) von einer Umgebung des Öffnungsabschnitts (21) jenseits der Einführungsposition bis zu der Position näher an dem Schulterabschnitt (19) aufgetragen wurde.

11. Spritzenzylinder (12) nach Anspruch 10,
wobei das Silikonöl (27) bis zu einer Umgebung des Schulterabschnitts (19) aufgetragen wurde.

12. Spritzenzylinder (12) nach einem der Ansprüche 9 bis 11,
wobei ein Durchschnittswert der Abdeckung von einer Umgebung des Schulterabschnitts (19) bis zu der Einführungsposition 10 % oder weniger beträgt.

## Revendications

1. Procédé de fabrication d'un corps de seringue (12), comprenant :
une étape de fourniture consistant à fournir le corps de seringue (12) incluant une portion de corps (18) creux et étant configuré pour qu'un joint (16) coulissant sur une surface interne (18a) de la portion de corps (18) y soit inséré ;
une étape d'application consistant à appliquer une huile de silicone (27) non réactive ayant une viscosité de 3000 cps à 30 000 cps sur la surface interne (18a) par pulvérisation avec un taux de quantité de revêtement de 0,02 mg/cm² à 0,2 mg/cm² ; et
une étape de chauffage consistant à chauffer l'huile de silicone (27) appliquée sur la surface interne (18a) sans étalement ni lissage, et qui est chauffée en réalisant une stérilisation en l'autoclave sur le corps de seringue (12) pour réduire la viscosité de façon à permettre à l'huile de silicone de s'étaler dans une direction de la surface interne (18a) de la portion de corps (18) et des directions de gravité, l'huile de silicone (27) étant appliquée sur la surface interne (18a) dans un état où un liquide ne remplit pas le corps de seringue (12).

2. Procédé de fabrication du corps de seringue (12) selon la revendication 1, comprenant en outre
une étape d'obtention d'un emballage (72) en stockant le corps de seringue (12) dans le sachet de stérilisation (46).

3. Procédé de fabrication du corps de seringue (12) selon la revendication 1 ou 2,
dans lequel l'étape de chauffage est réalisée dans un état où un axe du corps de seringue (12) est sensiblement vertical.

4. Procédé de fabrication du corps de seringue (12) selon la revendication 3,
dans lequel, dans l'étape d'application, l'huile de silicone (27) est appliquée sur la surface interne (18a) en pulvérisant l'huile de silicone (27) d'un côté d'extrémité vers l'autre côté d'extrémité du corps de seringue (12), et
l'étape de chauffage est réalisée dans un état où l'autre côté d'extrémité est orienté vers le bas.

5. Procédé de fabrication du corps de seringue (12) selon l'une quelconque des revendications 1 à 4,
dans lequel, dans l'étape d'application, l'huile de silicone (27) est pulvérisée d'un côté d'extrémité vers l'autre côté d'extrémité du corps de seringue (12), tandis que de l'air est aspiré à l'intérieur du corps de seringue (12) depuis l'autre côté d'extrémité.

6. Procédé de fabrication du corps de seringue (12) selon la revendication 5,
dans lequel le corps de seringue (12) inclut : une portion d'ouverture (21) pour insérer le joint (16) au niveau dudit un côté d'extrémité ; et une buse (20) ayant un diamètre intérieur plus petit que le diamètre intérieur de la portion de corps (18) au niveau dudit autre côté d'extrémité.

7. Procédé de fabrication d'une seringue préremplie, comprenant :
une étape de préparation consistant à préparer le corps de seringue (12) fabriqué par le procédé de fabrication du corps de seringue (12) selon l'une quelconque des revendications 1 à 6 ;
une étape de remplissage consistant à remplir le corps de seringue (12) préparé avec une solution médicamenteuse ; et
une étape de bouchage consistant à entrer le joint (16) dans le corps de seringue (12) rempli de la solution médicamenteuse pour le boucher.

8. Procédé de fabrication d'une seringue préremplie selon la revendication 7,
dans lequel la solution médicamenteuse est une préparation protéique.

9. Corps de seringue (12) comprenant : une portion de corps (18) creux ; une portion d'épaulement (19) réduite en diamètre à partir d'une extrémité distale de la portion de corps (18) ; une buse (20) faisant saillie dans une direction d'extrémité distale à partir de la portion d'épaulement (19) ; et une portion d'ouverture (21) qui s'ouvre à une extrémité proximale de la portion de corps (18), le corps de seringue (12) étant configuré pour qu'un joint (16) coulissant sur une surface interne (18a) de la portion de corps (18) y soit inséré,
dans lequel une huile de silicone (27) non réactive ayant une viscosité de 3000 cps à 30 000 cps a été appliquée sur la surface interne au moins à partir d'une position d'insertion (P) configurée pour que le joint (16) y soit inséré, vers une position plus proche de la portion d'épaulement (19) que la position d'insertion (P), et une couverture en tant que rapport entre l'aire de l'huile de silicone (27) présente sous forme de gouttelettes et l'aire de la surface interne (18a) est de 20 % ou moins à chacune des sections où une région appliquée avec l'huile de silicone (27) dans la surface interne (18a) est partitionnée d'une largeur prédéterminée dans la direction axiale, et une structure de gouttelette de l'huile de silicone (27) est présente sur la surface interne (18a).

10. Corps de seringue (12) selon la revendication 9,
dans lequel l'huile de silicone (27) a été appliquée depuis un voisinage de la portion d'ouverture (21) au-delà de la position d'insertion jusqu'à la position plus proche de la portion d'épaulement (19).

11. Corps de seringue (12) selon la revendication 10, dans lequel l'huile de silicone (27) a été appliquée jusqu'à un voisinage de la portion d'épaulement (19).

12. Corps de seringue (12) selon l'une quelconque des revendications 9 à 11,
dans lequel une valeur moyenne de la couverture depuis un voisinage de la portion d'épaulement (19) jusqu'à la position d'insertion est de 10 % ou moins.
